# EUROPEAN PATENT SPECIFICATION

(11) **EP 3 104 792 B1**
(45) Date of publication and mention of the grant of the patent: **15.06.2022**
(21) Application number: 14882554.0
(22) Date of filing: 05.11.2014
(51) Int. Cl.: A61B 34/00, A61B 34/30, A61B 17/29

(54) **SURGICAL END EFFECTORS AND PULLEY ASSEMBLIES THEREOF**
CHIRURGISCHE ENDEFFEKTOREN UND RIEMENSCHEIBENANORDNUNGEN DAVON
EFFECTEURS FINAUX CHIRURGICAUX ET LEURS ENSEMBLES POULIES

(30) Priority: 12.02.2014 US 201461938728 P
(43) Date of publication of application: 21.12.2016
(73) Proprietor: Covidien LP, Mansfield, MA 02048 (US)
(72) Inventor: SEOW, Chi Min, New Haven, Connecticut 06511 (US)
(74) Representative: Soames, Candida Jane
(86) International application number: PCT/US2014/064009
(87) International publication number: WO 2015/122944

(56) References cited:
- WO-A1-2011/115310
- WO-A1-2014/012780
- WO-A2-2009/051418
- KR-A- 20100 001 823
- KR-B1- 100 778 387
- US-A1- 2001 021 859
- US-A1- 2011 238 064
- US-A1- 2011 238 064
- US-B2- 8 617 203

## Description

### BACKGROUND

Robotic surgical systems have been used in minimally invasive medical procedures. Some robotic surgical systems included a console supporting a robot arm, and at least one end effector such as forceps or a grasping tool including jaws for capturing tissue therebetween. The at least one end effector was mounted to the robot arm. During a medical procedure, the end effector was inserted into a small incision (via a cannula) or a natural orifice of a patient to position the end effector at a work site within the body of the patient.

Cables extended from the console, through the robot arm, and connected to the end effector. In some instances, the cables were actuated by means of motors that were controlled by a processing system including a user interface for a surgeon or clinician to be able to control the robotic surgical system including the robot arm and/or the end effector. The cables connected to a pulley assembly that transferred torque to drive the actuation of the end effector.

In some instances, surgical procedures may require fine control of the end effector to grasp tissue for dissection and/or to spread tissue surfaces for deep tissue access. Accordingly, there is a need for surgical tools that are able to provide precisely controlled forces applied by jaws of an end effector of a robotic surgical system.

US2001/0021859 A1 discloses a forceps with two movable jaws and a pulley arrangement for activating the jaws, each jaw connected to a rotary pulley and the arrangement comprising a further pulley connected to a driving motor, the rotary pulleys are connected by driving wires to the pulley connected to the motor.

### SUMMARY

The invention is defined by independent claim 1 and further preferred embodiments are disclosed by the dependent claims. Jaws at the end of surgical robotics tools, such as forceps or scissor cutting tools, are driven by a pulley assembly including pulleys and cables. In accordance with an aspect of the present disclosure, an end effector of a surgical tool comprises a first jaw and a second jaw each being rotatable about a first axis. A first driven pulley is attached to the first jaw and a second driven pulley is attached to the second jaw. The first and second driven pulleys are rotatable about the first axis. Each driven pulley includes a first radial side and a second radial side. A driving pulley is rotatable about a second axis. A first cable has a first end portion, a second end portion, and an intermediate portion. The first end portion is connected to the first radial side of the first driven pulley. The second end portion is connected to the second radial side of the second driven pulley. The intermediate portion is connected to the driving pulley. A second cable has a first end portion, a second end portion, and an intermediate portion. The first end portion is connected to the first radial side of the second driven pulley. The second end portion is connected to the second radial side of the first driven pulley. The intermediate portion is connected to the driving pulley. A rotation of the driving pulley about the second axis rotates the driven pulleys in opposite directions about the first axis to open or close the jaws.

In some embodiments, a rotation of the driving pulley in a first direction about the second axis may rotate the first and second driven pulleys via the first cable. A rotation of the driving pulley in a second direction, opposite the first direction, may rotate the first and second driven pulleys via the second cable.

In aspects of the present disclosure, during rotation of the driving pulley in the first direction, the first cable may be in a tensioned condition and the second cable may be in a slack condition. During rotation of the driving pulley in the second direction, the first cable may be in a slack condition and the second cable may be in a tensioned condition.

In another aspect of the present disclosure, the intermediate portions of the first and second cables may be connected to a common point of the driving pulley or may be connected to different points of the driving pulley. It is contemplated that the intermediate portions of the first and second cables may be crimped to the driving pulley.

According to the invention, the first end portion of the first cable and the second end portion of the second cable are parallel, and in other instances not according to the invention, the first end portion of the second cable and the second end portion of the first cable may cross. In other instances not according to the invention, the respective end portions of the cables may be perpendicular instead of parallel or may be positioned at different angles that are neither parallel nor perpendicular.

In some embodiments, a proximal end of the first jaw may be fixedly attached to a circumferential edge of the first drive pulley and a proximal end of the second jaw may be fixedly attached to a circumferential edge of the second drive pulley.

In aspects of the present disclosure, the first and second jaws may be in flush engagement with one another. It is contemplated that the first axis may be spaced a lateral distance from the second axis. It is further contemplated that the first and second cables may be connected to the driving pulley at a location off-set a radial distance from the second axis.

In embodiments, the driving pulley may support an anchor member, and the intermediate portions of the first and second cables may each be looped through the anchor member of the driving pulley. The anchor member may include a hook that can be attached to a circumferential edge of the driving pulley.

In accordance with another aspect of the present disclosure, a pulley assembly for actuating a first jaw and a second jaw is provided. The pulley assembly comprises a first driven pulley configured to be attached to the first jaw and a second driven pulley configured to be attached to the second jaw. The first and second driven pulleys are rotatable about a first axis. Each driven pulley includes a first radial side and a second radial side. A driving pulley is rotatable about a second axis. A first cable has a first end portion, a second end portion, and an intermediate portion. The first end portion is connected to the first radial side of the first driven pulley. The second end portion is connected to the second radial side of the second driven pulley. The intermediate portion is connected to the driving pulley. A second cable has a first end portion, a second end portion, and an intermediate portion. The first end portion is connected to the first radial side of the second driven pulley. The second end portion is connected to the second radial side of the first driven pulley. The intermediate portion is connected to the driving pulley. A rotation of the driving pulley about the second axis rotates the first and second driven pulleys in opposite directions about the first axis.

In aspects of the present disclosure, a rotation of the driving pulley in a first direction about the second axis may rotate the first and second driven pulleys via the first cable. A rotation of the driving pulley in a second direction, opposite the first direction, may rotate the first and second driven pulleys via the second cable. In embodiments, during rotation of the driving pulley in the first direction, the first cable may be in a tensioned condition and the second cable may be in a slack condition. During rotation of the driving pulley in the second direction, the first cable may be in a slack condition and the second cable may be in a tensioned condition.

In some of these aspects, the intermediate portions of the first and second cables may be connected to a common point of the driving pulley or may be connected to different points of the driving pulley. In some instances, the first and second cables may be crimped to the driving pulley.

In some of the aforementioned aspects, the first end portion of the first cable and the second end portion of the second cable are parallel, and the first end portion of the second cable and the second end portion of the first cable cross. In other instances not according to the invention, the respective end portions of the cables may be perpendicular instead of parallel or may be positioned at different angles that are neither parallel nor perpendicular.

In accordance with yet another aspect of the present disclosure, another pulley assembly for actuating a first jaw and a second jaw is provided. The pulley assembly includes at least two driven pulleys. Each driven pulley may be coupled to a respective jaw and each driven pulley includes at least two radial sides.

The pulley assembly includes a driving pulley rotatable about a different axis from the driven pulleys and at least two cable sections. Each cable section may couple different radial sides of each driven pulley to the driving pulley. A directional change in rotation of the driving pulley may relieve a tension in at least two first cable sections coupled to different radial sides of at least two of the driven pulleys and may apply a tension to at least two second cable sections coupled to opposite radial sides of the driven pulleys than the first two cable sections.

In some instances, a first cable section is coupled between a first radial side of a first driven pulley and the driving pulley. A second cable section is coupled between a second radial side of the first driven pulley and the driving pulley. A third cable section is coupled between a first radial side of a second driven pulley and the driving pulley. A fourth cable section is coupled between a second radial side of the second driven pulley and the driving pulley.

The first and the fourth cable sections may be tensioned and the second and the third cable sections may be slackened when the driving pulley is rotated in a first direction. The first and the fourth cable sections may be slackened and the second and the third cable sections may be tensioned when the driving pulley is rotated in a second direction. In some instances, at least two of the cable sections may be part of a single continuous cable.

The first and the second cable sections are part of a first single continuous cable and the third and the fourth cable sections are part of a second single continuous cable. In other instances not forming part of the invention, the first and the fourth cable sections may be part of a first single continuous cable and the second and the third cable sections may be part of a second single continuous cable. In other instances not forming part of the invention, each of the cable sections may be a separate cable from the other cable sections. In other embodiments of the invention, two of the cable sections are part of a single continuous cable and two of the cable sections are separate cables.

Further details and aspects of embodiments of the invention and exemplary embodiments of the present disclosure are described in more detail below with reference to the appended figures.

As used herein, the terms parallel and perpendicular are understood to include relative configurations that are substantially parallel and substantially perpendicular, such as up to about + or - 10 degrees from true parallel and true perpendicular.

### BRIEF DESCRIPTION OF THE DRAWINGS

Embodiments of the present disclosure are described herein with reference to the accompanying drawings, wherein:
FIG. 1A is a schematic illustration of a medical work station and operating console in accordance with the present disclosure;
FIG. 1B is a schematic, perspective view of a motor of a control device of the medical work station of FIG. 1A, having a cable connected thereto;
FIG. 2 is a schematic plan view, with parts separated, of a surgical end effector, according to an embodiment of the present disclosure, illustrating jaws and a pulley assembly thereof;
FIG. 3A is a perspective view of the pulley assembly of the end effector shown in FIG. 2;
FIG. 3B is a perspective view of an alternate pulley assembly to that shown in FIG. 3A, the alternate pulley assembly of Fig 3B is not part of the present invention;
FIG. 4 is a perspective, cutaway view of the end effector shown in FIG. 2 with the jaws disposed in a closed configuration;
FIG. 5 is a perspective, cutaway view of the end effector shown in FIG. 2 with the jaws disposed in an open configuration; and
FIG. 6 is a schematic plan view, with parts separated, of a surgical end effector, according to another embodiment of the present disclosure, illustrating jaws and a pulley assembly thereof, however not forming part of the present invention.

### DETAILED DESCRIPTION

Embodiments of the presently disclosed surgical end effectors and exemplary methods of actuating the same are described in detail with reference to the drawings, in which like reference numerals designate identical or corresponding elements in each of the several views. The methods do not form part of the present invention. As used herein the term "distal" refers to that portion of the jaws and/or pulley assembly that is closer to a surgical site, while the term "proximal" refers to that portion of the jaws and/or pulley assembly that is farther from the surgical site.

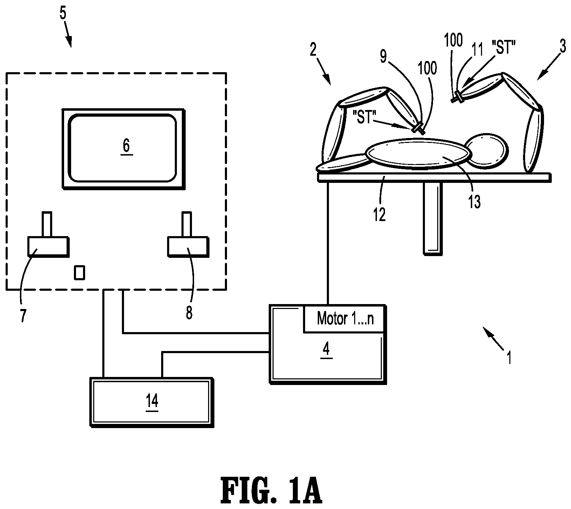

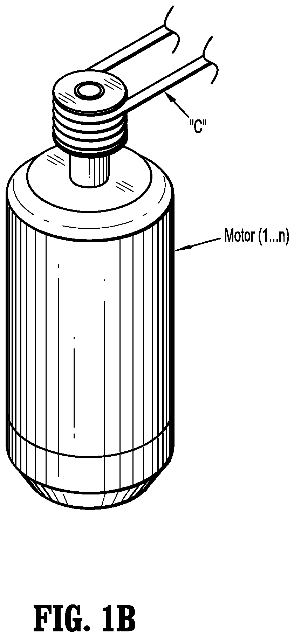

Referring initially to FIGS. 1A and 1B, a medical work station is shown generally as work station 1 and may include one or more robot arms 2, 3; a control device 4; and an operating console 5 coupled with control device 4. Operating console 5 includes a display device 6, which is set up in particular to display three-dimensional images and/or video; and manual input devices 7, 8, by means of which a person (not shown), for example a surgeon, is able to telemanipulate robot arms 2, 3 in a first operating mode, as known in principle to a person skilled in the art.

Each of the robot arms 2, 3 includes an attaching device 9, 11, to which may be attached, for example, a surgical tool "ST" supporting an end effector 100, in accordance with any one of several embodiments disclosed herein, as will be described in greater detail below.

Robot arms 2, 3 may be driven by electric drives (not shown) that are connected to control device 4. Control device 4 (e.g., a computer) is set up to activate the drives, in particular by means of a computer program, in such a way that robot arms 2, 3, their attaching devices 9, 11 and thus the surgical tool (including end effector 100) execute a desired movement according to a movement defined by means of manual input devices 7, 8. Control device 4 may also be set up in such a way that it regulates the movement of robot arms 2, 3 and/or of the drives.

Medical work station 1 is configured for use on a patient 13 lying on a patient table 12 to be treated in a minimally invasive manner by means of end effector 100. Medical work station 1 may also include one or more robot arms 2, 3, the additional robot arms likewise being connected to control device 4 and being telemanipulatable by means of operating console 5. A medical instrument or surgical tool (including an end effector 100) may also be attached to the additional robot arm. Medical work station 1 may include a database 14, in particular coupled to with control device 4, in which are stored for example pre-operative data from patient 13 and/or anatomical atlases.

Reference may be made to U.S. Patent Publication No. 2012/0116416, filed on November 3, 2011, entitled "Medical Workstation,", for a detailed discussion of the construction and operation of medical work station 1.

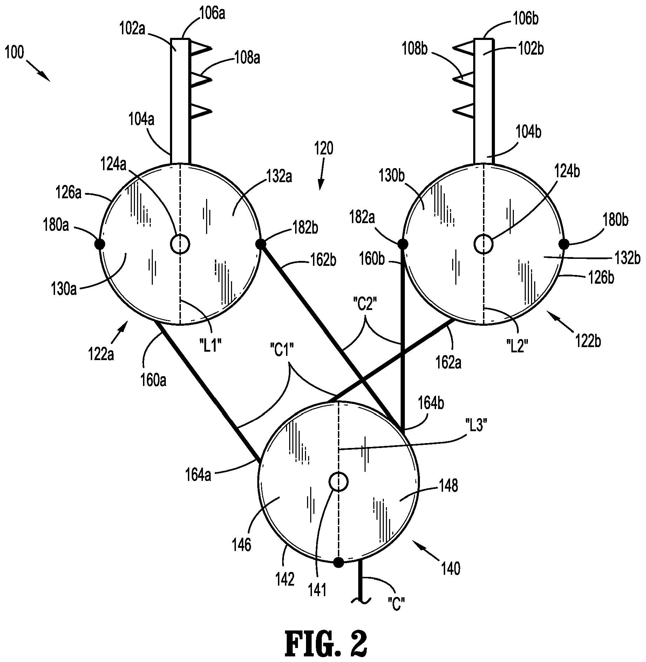

Control device 4 may control a plurality of motors (Motor 1...n) with each motor configured to wind-up or let out a length of cable "C" (FIG. 1B) extending to end effector 100 of the surgical tool. The distal end of each cable "C" is wrapped around a driving pulley 140 of end effector 100 in the manner of a capstan to drive a rotation of driving pulley 140 as shown, for example, in FIG. 2. In use, as cables "C" are wound-up and let out, cables "C" effect operation and/or movement of each end effector 100 of the surgical tool via pulley assembly 120, as described in further detail herein below. It is contemplated that control device 4 coordinates the activation of the various motors (Motor 1...n) to coordinate a winding-up or letting out a length of a respective cable "C" in order to coordinate an operation and/or movement of a respective end effector. Although FIG. 1B shows a single cable "C" that is wound up or let out by a single motor, in some instances two or more cables or two ends of a single cable may be wound up or let out by a single motor. For example, in some instances, two cables or cable ends may be coupled in opposite directions to a single motor so that as the motor is activated in a first direction, one of the cables winds up while the other cable lets out. Other cable configurations may be used in different embodiments.

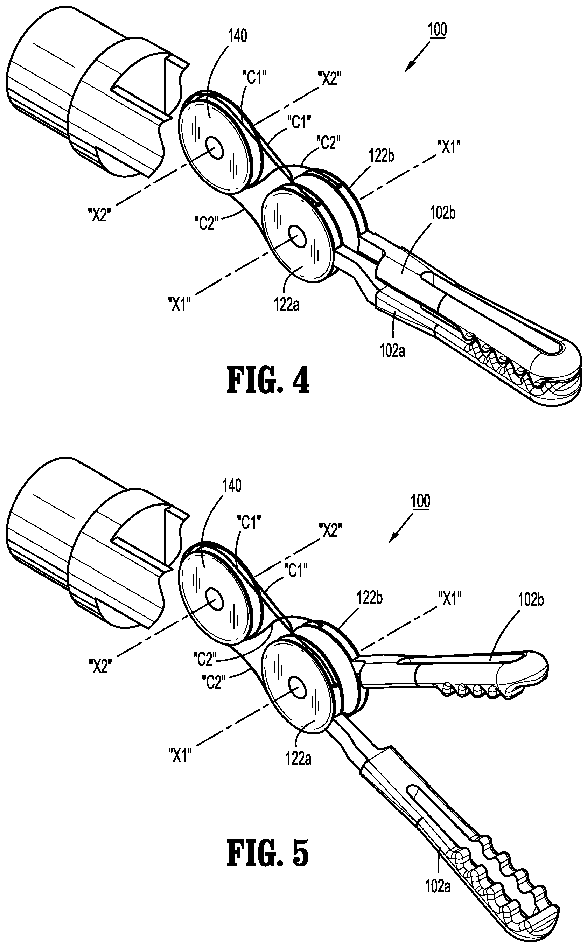

Turning now to FIGS. 2-5, an end effector in accordance with an embodiment of the present disclosure is generally designated as 100. End effector 100 includes a first jaw 102a and a second jaw 102b. First and second jaws 102a, 102b are each rotatable or pivotable relative to one another. Each jaw 102a, 102b has a respective proximal end 104a, 104b and a respective distal end 106a, 106b. Each proximal end 104a, 104b is fixedly attached to first and second driven pulleys 122a, 122b, respectively, as described in further detail herein below. Each proximal end 104a, 104b of jaws 102a, 102b can be integrally connected to and/or monolithically formed with a circumferential edge of driven pulleys 122a, 122b, respectively. Each distal end 106a, 106b of jaws 102a, 102b defines a respective grip or toothed portion 108a, 108b in juxtaposed relation to one another. In use, as will be described in greater detail below, as driving pulley 140 is rotated in one of a clockwise and counter clockwise direction, jaws 102a, 102b will be caused to rotate, moving jaws 102a, 102b from a first, open configuration in which jaws 102a, 102b may receive tissue therebetween to a second, closed configuration in which jaws 102a, 102b may grasp tissue.

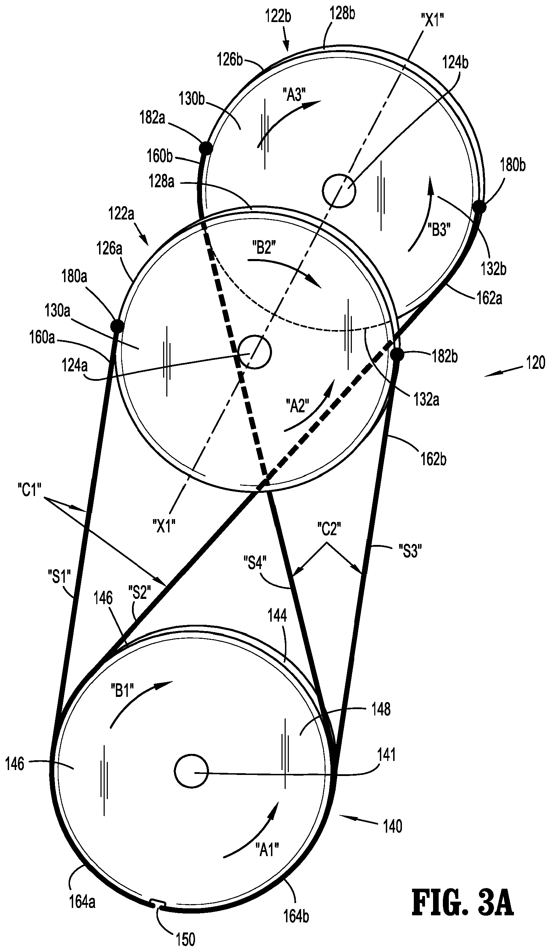

End effector 100 includes a pulley assembly 120 disposed therein for actuating jaws 102a, 102b of end effector 100. Pulley assembly 120 includes a first driven pulley 122a, a second driven pulley 122b, a driving pulley 140, a first cable "C1" and a second cable "C2." In FIG. 3A, cables C1 and C2 are each continuous cables that may have different cable sections, such as section S1 on cable C1 running from anchor member 180a on driven pulley 122a to anchor member 150 on driving pulley 140 and section S2 on cable C1 running from anchor member 150 to anchor member 180b on driven pulley 122b. Cable C2 may include cable sections S3 running from anchor member 182b on driven pulley 122a to anchor member 150 and section S4 running from anchor member 150 to anchor member 182a on driven pulley 122b.

In another embodiment that is a variation of that shown in FIG. 3A, cables C1 and C2 may also be continuous cables that may be attached to pulleys 122a, 122b, and 140 in a different manner. For example, continuous cable C1 may include a first section running from anchor member 180a on driven pulley 122a to anchor member 150 on driving pulley 140 (similar to section S1 in FIG. 3A) and a second section running from anchor member 150 to anchor member 182a on driven pulley 122b (similar to section S4 in FIG. 3A). Continuous cable C2 may include a first section running from anchor member 180b on driven pulley 122b to anchor member 150 (similar to section S2 in FIG. 3A) and a second section running from anchor member 150 to anchor member 182b on driven pulley 122a (similar to section S3 in FIG. 3A).

This configuration may result in a tensioning of a first section of cables C1 and C2 during a rotation of the driving pulley 140 in a first direction as well as a slacking of the other second section of cables C1 and C2. A tensioning of the second sections of cables C1 and C2 and a slacking of the first sections of cables C1 and C2 may occur when rotating the driving pulley 140 in the opposite direction. Other cable routings may be possible in different embodiments.

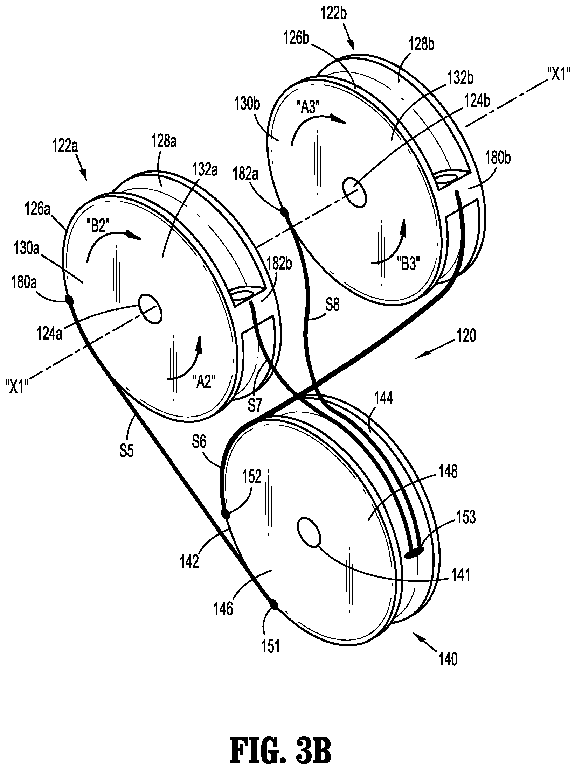

FIG. 3B shows an example not forming part of the present invention, in which continuous cables C1 and C2 are replaced with four non-continuous sections of cable S5 to S8. In FIG. 3B, cable section S5 runs from anchor member 180a on driven pulley 122a to anchor member 151 on driving pulley 140. Cable section S6 runs from a different anchor member 152 on driving pulley 140 to anchor member 180b on driven pulley 122b. Cable section S7 runs from anchor member 182b on driven pulley 122a to anchor member 153 on driving pulley 140. Cable section S8 runs from anchor member 153 on driving pulley 140 to anchor member 182a on driven pulley 122b. Cables and/or cable sections may be connected to the same or different anchor members on the driving pulley 140 in different embodiments.

In embodiments, jaws 102a, 102b may be detachably engaged to driven pulleys 122a, 122b via a hinge, clips, buttons, adhesives, ferrule, snap-fit, threaded, and/or other engagement.

Each driven pulley 122a, 122b has a central opening 124a, 124b formed therein configured for disposal or receipt of a pivot pin (not shown) therein. Central openings 124a, 124b of each driven pulley 122a, 122b are in coaxial alignment with one another. A first axis "XI" extends through central openings 124a, 124b of first and second driven pulleys 122a, 122b. First and second driven pulleys 122a, 122b are disposed adjacent to one another and are rotatable relative to one another about first axis "X1." In some embodiments, driven pulleys 122a, 122b may be in abutting relation to one another or in spaced apart relation to one another, along first axis "X1." As mentioned above, first driven pulley 122a supports jaw 102a and second driven pulley 122b supports jaw 102b such that jaws 102a, 102b rotate with driven pulleys 122a, 122b about first axis "X1."

Driven pulleys 122a, 122b have a circular configuration and each define a circumferential edge 126a, 126b. Circumferential edges 126a, 126b each define an arcuate channel or groove 128a, 128b extending along a circumference of each driven pulley 122a, 122b. Channel or groove 128a, 128b is configured for receipt of one of cables "C1," "C2," as described in further detail herein below. In embodiments, driven pulleys 122a, 122b are variously configured, such as, for example, oval, oblong, tapered, arcuate, uniform, non-uniform and/or variable.

First driven pulley 122a includes a first radial side 130a and a second radial side 132a each defining a semicircular portion of first driven pulley 122a, as demarcated by dotted line "L1" in FIG. 2. First and second radial sides 130a, 132a each include one-half of circumferential edge 126a of first driven pulley 122a. Second driven pulley 122b includes a first radial side 130b and a second radial side 132b each defining a semicircular portion of second driven pulley 122b, as demarcated by dotted line "L2" in FIG. 2. First and second radial sides 130b, 132b of second driven pulley 122b include one-half of circumferential edge 126b of second driven pulley 122b.

Pulley assembly 120 further includes a driving pulley 140, similar to first and second driven pulleys 122a, 122b described herein above. Driving pulley 140 is spaced a lateral distance from first and second driven pulleys 122a, 122b. Cable "C," connected to motor (Motor 1....n), may be wrapped at least once around driving pulley 140, in the manner of a capstan so as to not interfere with first and second cables "C1" "C2." Driving pulley 140 includes a central opening 141 formed therein. A second axis "X2" passes through central opening 141, is spaced a lateral distance from first axis "X1," and may run parallel to first axis "XI" in some instances. In other instances, the second axis "X2" may be offset from the first axis "XI" so that it runs at other non-parallel angles to the first axis "X1," such as perpendicular to the first axis.

Driving pulley 140 has a circular configuration and defines a circumferential edge 142. Circumferential edge 142 defines an arcuate channel or groove 144 extending along a circumference of driving pulley 140. Channel or groove 144 is configured for disposal of each of cables "C1," "C2." Driving pulley 140 includes a first radial side 146 and a second radial side 148 each defining a semicircular portion of driving pulley 140, as demarcated by dotted line "L3" in FIG. 2. First and second radial sides 146, 148 each include one-half of circumferential edge 142 of driving pulley 140.

Driving pulley 140 supports an anchor member 150 attached to a proximal-most portion of circumferential edge 142. Anchor member 150 secures both cables "C1," "C2" to drive pulley 140 such that, as driving pulley 140 is rotated, cables "C1," "C2" move therewith. In embodiments, anchor member 150 may be a hook onto which cables "C1," "C2" are attached. In other embodiments, anchor member 150 may be a crimp that secures cables "C1" "C2" to circumferential edge 142 of driving pulley 140.

In use, a rotation of driving pulley 140 about second axis "X2" via motor (Motor 1...n) and cable "C" causes first and second driven pulleys 122a, 122b to rotate, via cables "C1" "C2," in opposing directions about first axis "XI" to open or close first and second jaws 102a, 102b, which are attached thereto.

In an embodiment of the invention, pulley assembly 120 further includes a first cable "C1" and a second cable "C2." First cable "C1" and second cable "C2" each have a first end portion 160a, 160b, a second end portion 162a, 162b, and an intermediate portion or looped portion 164a, 164b. First and second cables "C1" "C2" are connected to first and second driven pulleys 122a, 122b and driving pulley 140 such that first end portion 160a of first cable "C1" and second end portion 162b of second cable "C2" are substantially parallel, and first end portion 160b of second cable "C2" and second end portion 162a of first cable "C1" cross, as shown in FIGS 2-5.

First cable "C1" is secured by anchor member 150 of driving pulley 140 to a proximal-most portion of circumferential edge 142 of driving pulley 140 such that intermediate portion or looped portion 164a of first cable "C1" is fixedly engaged with a portion of circumferential edge 142 of driving pulley 140. Intermediate portion or looped portion 164a of first cable "C1" is connected to driving pulley 140 at a location off-set a radial distance from second axis "X2."

First end portion 160a of first cable "C1" is connected to a portion of circumferential edge 126a of first driven pulley 122a that is disposed on first radial side 130a of first driven pulley 122a. Second end portion 162a of first cable "C1" is connected to a portion of circumferential edge 126b of second driven pulley 122b that is disposed on second radial side 132b of second driven pulley 122b.

First end portion 160a of first cable "C1" is connected to first radial side 130a of first driven pulley 122a via an anchor member 180a. Second end portion 162a of first cable "C1" is connected to second radial side 132b of second driven pulley 122b via an anchor member 180b. Anchor members 180a, 180b are similar to anchor member 150 described above. Each anchor member 150, 180a, 180b can be the same or may be different. In this way, intermediate portion or looped portion 164a of first cable "C1" is wrapped around only first radial side 146 of driving pulley 140, as shown in FIGS. 3A, 4, and 5.

Second cable "C2" is secured by anchor member 150 of driving pulley 140 to a proximal-most portion of circumferential edge 142 of driving pulley 140 such that intermediate portion or looped portion 164b of second cable "C2" is fixedly engaged with a portion of circumferential edge 142 of driving pulley 140. Intermediate portion or looped portion 164b of second cable "C2" is connected to driving pulley 140 at a location off-set a radial distance from second axis "X2." In this way, intermediate portions or looped portions 164a, 164b of first and second cables "C1," "C2" are connected to a common point of driving pulley 140.

First end portion 160b of second cable "C2" is connected to a portion of circumferential edge 126b of second driven pulley 122b that is disposed on first radial side 130b of second driven pulley 122b. Second end portion 162b of second cable "C2" is connected to a portion of circumferential edge 126a of first driven pulley 122a that is disposed on second radial side 132a of first driven pulley 122a.

First end portion 160b of second cable "C2" is connected to first radial side 130b of second driven pulley 122b via an anchor member 182a. Second end portion 162b of second cable "C2" is connected to second radial side 132a of first driven pulley 122a via an anchor member 182b. Anchor members 182a, 182b are similar to anchor member 150 described above. In this way, intermediate portion 164b of second cable "C2" is wrapped around only second radial side 148 of driving pulley 140, as shown in FIGS. 3-5.

In one embodiment, first cable "C1" includes two cables each having a first end connected to driving pulley 140 at a common point and a second end connected to first radial side 130a of first driven pulley 122a and second radial side 132b of second driven pulley 122b, respectively. In a further embodiment, second cable "C2" includes two cables each having a first end connected to driving pulley 140 at a common point and a second end connected to first radial side 130b of second driven pulley 122b and second radial side 132a of first driven pulley 122a, respectively.

In operation, motor (Motor 1...n) is energized to rotate and, in turn, drive a letting out or winding-up or a rotation of cable "C." As cable "C" is actuated, cable "C" drives the rotation of driving pulley 140 in one of a clockwise and counter-clockwise direction. A rotation of driving pulley 140 in a first direction, indicated by arrow "A1" shown in FIG. 3A, about second axis "X2," rotates first and second driven pulleys 122a, 122b via first cable "C1" about first axis "X1," in a direction indicated by arrows "A2," "A3" in FIG. 3A, respectively. During rotation of driving pulley 140 in the first direction, first cable "C1" is in a tensioned condition (shown in FIG. 3A) and second cable "C2" is in a slack condition. For example, as driving pulley 140 is rotated in the first direction, intermediate portion 164a of first cable "C1" rotates with driving pulley 140 about second axis "X2." As intermediate portion 164a of first cable "C1" rotates, first end portion 160a of first cable "C1" is pulled towards driving pulley 140 and, in turn, drives a rotation of first driven pulley 122a in the same direction as the direction in which driving pulley 140 is rotating. Second end portion 162a of first cable "C1" is also pulled towards driving pulley 140 and, in turn, drives a rotation of second driven pulley 122b in an opposite direction as the direction in which driving pulley 140 is rotating. In this way, jaws 102a, 102b, which are attached to driven pulleys 122a, 122b, respectively, are opened about first axis "X1."

A rotation of driving pulley 140 in a second direction, indicated by arrow "B1" shown in FIG. 3A, rotates first and second driven pulleys 122a, 122b via second cable "C2" about first axis "X1," in a direction indicated by arrows "B2," "B3" in FIG. 3A, respectively. During rotation of driving pulley 140 in the second direction, first cable "C1" is in a slack condition and second cable "C2" is in a tensioned condition (shown in FIG. 3A). For example, as driving pulley 140 is rotated in the second direction, intermediate portion 164b of second cable "C2" rotates with driving pulley 140 about second axis "X2." As intermediate portion 164b of second cable "C2" rotates, first end portion 160b of second cable "C2" is pulled towards driving pulley 140 and, in turn, drives a rotation of second driven pulley 122b in an opposite direction as the direction in which driving pulley 140 is rotating. Second end portion 162b of second cable "C2" is also pulled towards driving pulley 140 and, in turn, drives a rotation of first driven pulley 122a in the same direction in which driving pulley 140 is rotating. In this way, jaws 102a, 102b, which are attached to driven pulleys 122a, 122b, respectively, are closed about first axis "X1."

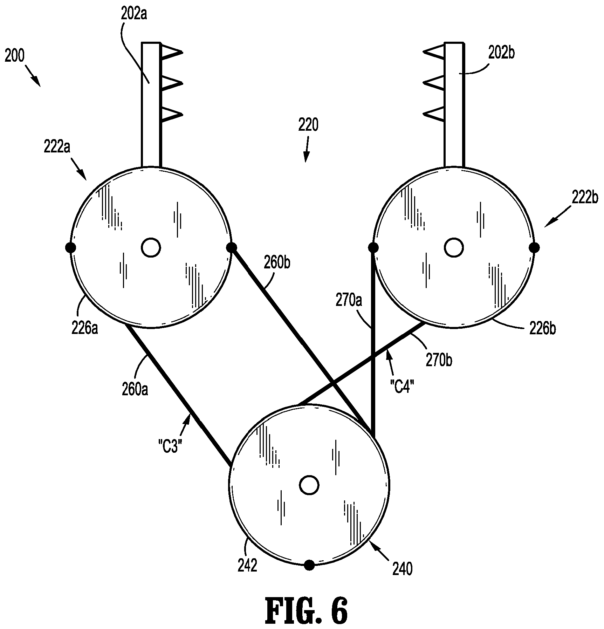

In a further example not forming part of the invention, as shown in FIG. 6, an end effector 200, similar to end effector 100 described above with regard to FIGS. 2-5, is shown. End effector 200 includes a first jaw 202a and a second jaw 202b, similar to jaws 102a, 102b described above. First and second jaws 202a, 202b are each pivotable about a first axis (not shown). End effector 200 further includes a pulley assembly 220, similar to pulley assembly 120 described above. Pulley assembly 220 is disposed within end effector 200 for actuating jaws 202a, 202b of end effector 200.

Pulley assembly 220 includes a first driven pulley 222a, a second driven pulley 222b, a driving pulley 240, a first cable "C3," and a second cable "C4," similar to first driven pulley 122a, second driven pulley 122b, driving pulley 140, first cable "C1" and second cable "C2," respectively, described above. In accordance with the present embodiment, first cable "C3" and second cable "C4" may be in the form of a cable loop or the like.

First driven pulley 222a supports jaw 202a and second driven pulley 222b supports jaw 202b such that jaws 202a, 202b rotate with driven pulleys 222a, 222b about the first axis. Driven pulleys 222a, 222b have a circular configuration and each define a circumferential edge 226a, 226b configured for disposal or receipt of first and second cables "C3," "C4," respectively.

Driving pulley 240 is spaced a lateral distance from first and second driven pulleys 222a, 222b. Cable "C," connected to motor (Motor 1....n), may be wrapped at least once around driving pulley 240, in the manner of a capstan so as to not interfere with first and second cables "C3," "C4." Driving pulley 240 has a circular configuration and defines a circumferential edge 242 configured for disposal or receipt of each of cables "C3," "C4."

First cable "C3" is looped or wrapped about circumferential edge 242 of driving pulley 240 and circumferential edge 226a of first driven pulley 222a such that, a first half 260a and a second half 260b of cable "C3" are in parallel relation to one another. Second cable "C4" is looped or wrapped about circumferential edge 242 of driving pulley 240 and circumferential edge 226b of second driven pulley 222b such that, a first half 270a and a second half 270b of cable "C4" are in a criss-cross or figure-eight pattern.

In use, a rotation of driving pulley 240 via motor (Motor 1...n) and cable "C" causes first and second driven pulleys 222a, 222b to rotate, via cables "C3," "C4," in opposing directions to open or close first and second jaws 202a, 202b, which are attached thereto.

It will be understood that various modifications may be made to the embodiments disclosed herein. For example, while the driven pulleys disclosed herein have been shown and described as being connected to the proximal ends of the jaws, it is contemplated and within the scope of the present disclosure, for the driven pulleys to be operatively connected with the distal portion of the jaws. Therefore, the above description should not be construed as limiting, but merely as exemplifications of various embodiments. Those skilled in the art will envision other modifications within the scope of the invention as defined by the claims appended thereto.

## Claims

1. An end effector (100) of a surgical tool, the end effector comprising:
a first jaw (102a) and a second jaw (102b) each being pivotable about a first axis ("X1");
a first driven pulley (122a) attached to the first jaw and a second driven pulley (122b) attached to the second jaw, the first and second driven pulleys being rotatable about the first axis, each driven pulley including a first radial side (130a, 130b) and a second radial side (132a, 132b);
a driving pulley (140) rotatable about a second axis ("X2");
a first cable section (S1) coupled between a first radial side of a first driven pulley and the driving pulley;
a second cable section (S3) coupled between a second radial side of the first driven pulley and the driving pulley;
a third cable section (S4) coupled between a first radial side of a second driven pulley and the driving pulley;
a fourth cable section (S2) coupled between a second radial side of the second driven pulley and the driving pulley;
wherein the first and the second cable sections (S1, S3) are parallel, and the third and the fourth cable sections (S4, S2) cross;
wherein a rotation of the driving pulley about the second axis rotates the first and second driven pulleys in opposite directions about the first axis to one of open and close the first and second jaws,
**characterised in that**:
(a) the first and the fourth cable sections (S1, S2) are part of a first single continuous cable (C1), and the second and the third cable sections (S3, S4) are part of a second single continuous cable (C₂); or
(b) the first and the fourth cable sections (S1, S2) are part of a first single continuous cable (C1), and the second and the third cable sections (S3, S4) are separate cables; or
(c) the first and the fourth cable sections (S1, S2) are separate cables, and the second and the third cable sections (S3, S4) are part of a second single continuous cable (C2).

2. The end effector (100) as recited in Claim 1 under the characterised feature (a), wherein a rotation of the driving pulley (140) in a first direction about the second axis ("X2") rotates the first and second driven pulleys (122a, 122b) via the first single continuous cable (C1), and a rotation of the driving pulley in a second direction, opposite the first direction, rotates the first and second driven pulleys via the second single continuous cable (C2).

3. The end effector (100) as recited in Claim 1 under the characterised feature (a) or Claim 2, wherein during rotation of the driving pulley (140) in the first direction the first single continuous cable (C1) is in a tensioned condition and the second single continuous cable (C2) is in a slack condition, and during rotation of the driving pulley in the second direction the first single continuous cable is in a slack condition and the second single continuous cable is in a tensioned condition.

4. The end effector (100) as recited in Claim 1 under the characterised feature (a) or any of Claims 2-3, wherein the first and second single continuous cables (C1, C2) further comprise an intermediate portion (164a, 164b) connected to the driving pulley (140).

5. The end effector (100) as recited in Claim 4, wherein the intermediate portions (164a, 164b) of the first and second single continuous cables (C1, C2) are connected to a common point of the driving pulley (140).

6. The end effector (100) as recited in Claim 4 or Claim 5, wherein the intermediate portions (164a, 164b) of the first and second single continuous cables (C1, C2) are crimped to the driving pulley (140).

7. The end effector (100) as recited in any preceding Claim, wherein a proximal end (104a) of the first jaw (102a) is fixedly attached to a circumferential edge (126a) of the first driven pulley (122a) and a proximal end (104b) of the second jaw (102b) is fixedly attached to a circumferential edge (126b) of the second driven pulley (122b).

8. The end effector (100) as recited in any preceding Claim, wherein the first and second jaws (102a, 102b) include a toothed portion (108a, 108b).

9. The end effector (100) as recited in any preceding Claim, wherein the first axis ("X1") is spaced a lateral distance from the second axis ("X2").

10. The end effector (100) as recited in Claim 1 under the characterised feature (a) or any of Claims 2-6, wherein the first and second single continuous cables (C1, C2) are connected to the driving pulley (140) at a location off-set a radial distance from the second axis ("X2").

11. The end effector (100) as recited in any of Claims 4-6, wherein the driving pulley (140) supports an anchor member (150), and the intermediate portions (164a, 164b) of the first and second single continuous cables (C1, C2) are each looped through the anchor member of the driving pulley.

12. The end effector (100) as recited in Claim 11, wherein the anchor member (150) includes a hook that is attached to a circumferential edge (142) of the driving pulley (140).

## Patentansprüche

1. Endeffektor (100) eines chirurgischen Instruments, wobei der Endeffektor Folgendes umfasst:
eine erste Backe (102a) und eine zweite Backe (102b), die jeweils um eine erste Achse ("X1") herum schwenkbar sind;
eine erste angetriebene Riemenscheibe (122a), die an der ersten Backe angebracht ist, und eine zweite angetriebene Riemenscheibe (122b), die an der zweiten Backe angebracht ist, wobei die erste und die zweite angetriebene Riemenscheibe um eine zweite Achse herum drehbar sind, wobei jede angetriebene Riemenscheibe eine erste radiale Seite (130a, 130b) und eine zweite radiale Seite (132a, 132b) einschließt;
eine antreibende Riemenscheibe (140), die um eine zweite Achse ("X2") herum drehbar ist;
einen ersten Kabelbereich (S1), der zwischen einer ersten radialen Seite einer ersten angetriebenen Riemenscheibe und der antreibenden Riemenscheibe gekoppelt ist;
einen zweiten Kabelbereich (S3), der zwischen einer zweiten radialen Seite der ersten angetriebenen Riemenscheibe und der antreibenden Riemenscheibe gekoppelt ist;
einen dritten Kabelbereich (S4), der zwischen einer ersten radialen Seite einer zweiten angetriebenen Riemenscheibe und der antreibenden Riemenscheibe gekoppelt ist;
einen vierten Kabelbereich (S2), der zwischen einer zweiten radialen Seite der zweiten angetriebenen Riemenscheibe und der antreibenden Riemenscheibe gekoppelt ist;
wobei der erste und der zweite Kabelbereich (S1, S3) parallel sind und sich der dritte und der vierte Kabelbereich (S4, S2) kreuzen;
wobei eine Drehung der antreibenden Riemenscheibe um die zweite Achse herum die erste und die zweite angetriebene Riemenscheibe in entgegengesetzten Richtungen um die erste Achse herum dreht, um die erste und die zweite Backe zu öffnen oder zu schließen,
**dadurch gekennzeichnet, dass**:
(a) der erste und der vierte Kabelbereich (S1, S2) Teil eines ersten einzelnen fortlaufenden Kabels (C1) sind und der zweite und der dritte Kabelbereich (S3, S4) Teil eines zweiten einzelnen fortlaufenden Kabels (C2) sind; oder
(b) der erste und der vierte Kabelbereich (S1, S2) Teil eines ersten einzelnen fortlaufenden Kabels (C1) sind und der zweite und der dritte Kabelbereich (S3, S4) getrennte Kabel sind; oder
(c) der erste und der vierte Kabelbereich (S1, S2) getrennte Kabel sind und der zweite und der dritte Kabelbereich (S3, S4) Teil eines zweiten einzelnen fortlaufenden Kabels (C2) sind.

2. Endeffektor (100) nach Anspruch 1 unter dem gekennzeichneten Merkmal (a), wobei eine Drehung der antreibenden Riemenscheibe (140) in einer ersten Richtung um die zweite Achse ("X2") herum die erste und die zweite angetriebene Riemenscheibe (122a, 122b) über das erste einzelne fortlaufende Kabel (C1) dreht und eine Drehung der antreibenden Riemenscheibe in einer zweiten Richtung, entgegen der ersten Richtung, die erste und die zweite angetriebene Riemenscheibe über das zweite einzelne fortlaufende Kabel (C2) dreht.

3. Endeffektor (100) nach Anspruch 1 unter dem gekennzeichneten Merkmal (a) oder Anspruch 2, wobei sich während der Drehung der antreibenden Riemenscheibe (140) in der ersten Richtung das erste einzelne fortlaufende Kabel (C1) in einem gespannten Zustand befindet und sich das zweite einzelne fortlaufende Kabel (C2) in einem durchhängenden Zustand befindet und sich während der Drehung der antreibenden Riemenscheibe in der zweiten Richtung das erste einzelne fortlaufende Kabel in einem durchhängenden Zustand befindet und sich das zweite einzelne fortlaufende Kabel in einem gespannten Zustand befindet.

4. Endeffektor (100) nach Anspruch 1 unter dem gekennzeichneten Merkmal (a) oder einem der Ansprüche 2-3, wobei das erste und das zweite einzelne fortlaufende Kabel (C1, C2) ferner einen Zwischenabschnitt (164a, 164b) umfassen, der mit der antreibenden Riemenscheibe (140) verbunden ist.

5. Endeffektor (100) nach Anspruch 4, wobei die Zwischenabschnitte (164a, 164b) des ersten und des zweiten einzelnen fortlaufenden Kabels (C1, C2) mit einem gemeinsamen Punkt der antreibenden Riemenscheibe (140) verbunden sind.

6. Endeffektor (100) nach Anspruch 4 oder 5, wobei die Zwischenabschnitte (164a, 164b) des ersten und des zweiten einzelnen fortlaufenden Kabels (C1, C2) auf die antreibende Riemenscheibe (140) gepresst sind.

7. Endeffektor (100) nach einem der vorhergehenden Ansprüche, wobei ein proximales Ende (104a) der ersten Backe (102a) an einem Umfangsrand (126a) der ersten angetriebenen Riemenscheibe (122a) fest angebracht ist und ein proximales Ende (104b) der zweiten Backe (102b) an einem Umfangsrand (126b) der zweiten angetriebenen Riemenscheibe (122b) fest angebracht ist.

8. Endeffektor (100) nach einem der vorhergehenden Ansprüche, wobei die erste und die zweite Backe (102a, 102b) einen gezahnten Abschnitt (108a, 108b) einschließen.

9. Endeffektor (100) nach einem der vorhergehenden Ansprüche, wobei die erste Achse ("X1") einen lateralen Abstand von der zweiten Achse ("X2") beabstandet ist.

10. Endeffektor (100) nach Anspruch 1 unter dem gekennzeichneten Merkmal (a) oder einem der Ansprüche 2-6, wobei das erste und das zweite einzelne fortlaufende Kabel (C1, C2) bei einer Lage versetzt um einen radialen Abstand von der zweiten Achse ("X2") mit der antreibenden Riemenscheibe (140) verbunden sind.

11. Endeffektor (100) nach einem der Ansprüche 4-6, wobei die antreibende Riemenscheibe (140) ein Ankerelement (150) trägt und die Zwischenabschnitte (164a, 164b) des ersten und des zweiten einzelnen fortlaufenden Kabels (C1, C2) jeweils durch das Ankerelement der antreibenden Riemenscheibe geschlungen sind.

12. Endeffektor (100) nach Anspruch 11, wobei das Ankerelement (150) einen Haken einschließt, der an einer Umfangskante (142) der antreibenden Riemenscheibe (140) angebracht ist.

## Revendications

1. Effecteur d'extrémité (100) d'un outil chirurgical, l'effecteur d'extrémité comprenant :
une première mâchoire (102a) et une seconde mâchoire (102b) pouvant chacune pivoter autour d'un premier axe (« X1 ») ;
une première poulie entraînée (122a) fixée à la première mâchoire et une seconde poulie entraînée (122b) fixée à la seconde mâchoire, les première et seconde poulies entraînées pouvant tourner autour du premier axe, chaque poulie entraînée comportant un premier côté radial (130a, 130b) et un second côté radial (132a, 132b) ;
une poulie d'entraînement (140), pouvant tourner autour d'un second axe (« X2 ») ;
une première section de câble (S1) accouplée entre un premier côté radial d'une première poulie entraînée et la poulie d'entraînement ;
une deuxième section de câble (S3) accouplée entre un second côté radial de la première poulie entraînée et la poulie d'entraînement ;
une troisième section de câble (S4) accouplée entre un premier côté radial d'une seconde poulie entraînée et la poulie d'entraînement ;
une quatrième section de câble (S2) accouplée entre un second côté radial de la seconde poulie entraînée et la poulie d'entraînement ;
dans lequel les première et deuxième sections de câble (S1, S3) sont parallèles, et les troisième et quatrième sections de câble (S4, S2) se croisent ;
dans lequel une rotation de la poulie d'entraînement autour du second axe fait tourner les première et seconde poulies entraînées dans des directions opposées autour du premier axe pour ouvrir ou fermer les première et seconde mâchoires,
**caractérisé en ce que** :
(a) les première et quatrième sections de câble (S1, S2) font partie d'un premier câble continu unique (C1), et les deuxième et troisième sections de câble (S3, S4) font partie d'un second câble continu unique (C2) ; ou
(b) les première et quatrième sections de câble (S1, S2) font partie d'un premier câble continu unique (C1), et les deuxième et troisième sections de câble (S3, S4) sont des câbles distincts ; ou
(c) les première et quatrième sections de câble (S1, S2) sont des câbles distincts, et les deuxième et troisième sections de câble (S3, S4) font partie d'un second câble continu unique (C2).

2. Effecteur d'extrémité (100) selon la revendication 1 sous l'élément caractérisé (a), dans lequel une rotation de la poulie d'entraînement (140) dans une première direction autour du second axe (« X2 ») fait tourner les première et seconde poulies entraînées (122a, 122b) par l'intermédiaire du premier câble unique continu (C1), et une rotation de la poulie d'entraînement dans une seconde direction, opposée à la première direction, fait tourner les première et seconde poulies entraînées par l'intermédiaire du second câble unique continu (C2).

3. Effecteur d'extrémité (100) selon la revendication 1 sous l'élément caractérisé (a) ou la revendication 2, dans lequel pendant la rotation de la poulie d'entraînement (140) dans la première direction, le premier câble continu unique (C1) est dans un état tendu et le second câble continu unique (C2) est dans un état lâche, et pendant la rotation de la poulie d'entraînement dans la seconde direction, le premier câble continu unique est dans un état lâche et le second câble continu unique est dans un état tendu.

4. Effecteur d'extrémité (100) selon la revendication 1 sous l'élément caractérisé (a) ou l'une quelconque des revendications 2 et 3, dans lequel les premier et second câbles continus uniques (C1, C2) comprennent en outre une partie intermédiaire (164a, 164b) reliée à la poulie d'entraînement (140).

5. Effecteur d'extrémité (100) selon la revendication 4, dans lequel les parties intermédiaires (164a, 164b) des premier et second câbles continus uniques (C1, C2) sont reliées au niveau d'un point commun de la poulie d'entraînement (140).

6. Effecteur d'extrémité (100) selon la revendication 4 ou 5, dans lequel les parties intermédiaires (164a, 164b) des premier et second câbles continus uniques (C1, C2) sont serties sur la poulie d'entraînement (140).

7. Effecteur d'extrémité (100) selon l'une quelconque des revendications précédentes, dans lequel une extrémité proximale (104a) de la première mâchoire (102a) est fixée de manière fixe à un bord circonférentiel (126a) de la première poulie entraînée (122a) et une extrémité proximale (104b) de la seconde mâchoire (102b) est fixée de manière fixe à un bord circonférentiel (126b) de la seconde poulie entraînée (122b).

8. Effecteur d'extrémité (100) selon l'une quelconque des revendications précédentes, dans lequel les première et seconde mâchoires (102a, 102b) comportent une partie dentée (108a, 108b).

9. Effecteur d'extrémité (100) selon l'une quelconque des revendications précédentes, dans lequel le premier axe (« X1 ») est espacé d'une distance latérale par rapport au second axe (« X2 »).

10. Effecteur d'extrémité (100) selon la revendication 1 sous l'élément caractérisé (a) ou l'une quelconque des revendications 2 à 6, dans lequel les premier et second câbles continus uniques (C1, C2) sont reliés à la poulie d'entraînement (140) au niveau d'un emplacement décalé d'une distance radiale par rapport au second axe (« X2 »).

11. Effecteur d'extrémité (100) selon l'une quelconque des revendications 4 à 6, dans lequel la poulie d'entraînement (140) supporte un élément d'ancrage (150) et les parties intermédiaires (164a, 164b) des premier et second câbles uniques continus (C1, C2) sont chacune bouclées à travers l'élément d'ancrage de la poulie d'entraînement.

12. Effecteur d'extrémité (100) selon la revendication 11, dans lequel l'élément d'ancrage (150) comporte un crochet qui est attaché à un bord circonférentiel (142) de la poulie d'entraînement (140).
